# EUROPEAN PATENT SPECIFICATION

(11) **EP 2 651 371 B1**
(45) Date of publication and mention of the grant of the patent: **27.06.2018**
(21) Application number: 11794188.0
(22) Date of filing: 13.12.2011
(51) Int. Cl.: A61K 8/04, A61K 8/81, A61Q 5/06, A61K 8/34

(54) **FAN-SHAPED AEROSOL DEVICE FOR STYLING THE HAIR**
AEROSOL FÜR EIN FÄCHERARTIGES VERSPRÜHEN EINER HAARSTYLINGZUSAMMENSETZUNG
DISPOSITIF AÉROSOL EN FORME D'ÉVENTAIL POUR COIFFER LES CHEVEUX

(30) Priority: 14.12.2010 FR 1060501; 15.02.2011 US 201161442899 P
(43) Date of publication of application: 23.10.2013
(73) Proprietor: L'Oréal, 75008 Paris (FR)
(72) Inventor: RODRIGUES, Céférino, F-93100 Montreuil (FR); MEDARD, Fatma, F-91220 Brétigny-sur-Orge (FR); GAWTREY, Jonathan, F-92100 Boulogne (FR)
(74) Representative: Crest, Véronique
(86) International application number: PCT/EP2011/072617
(87) International publication number: WO 2012/080255

(56) References cited:
- WO-A1-98/52517
- FR-A1- 2 708 908
- GB-A- 1 113 116
- US-A- 2 734 774
- US-A- 3 178 120
- US-A- 4 871 529
- US-A- 5 702 692
- US-A1- 2004 141 926

## Description

The present invention relates to an aerosol device for styling keratin fibres such as the hair, formed from a container containing a particular aerosol composition and a particular means for dispensing the said aerosol composition.

The present invention also relates to a treatment process, and especially a process for shaping the hairstyle in which this device is used, and also to the use of the said device for shaping keratin fibres, such as the hair.

Fixing of the hairstyle is an important element of styling, which consists in maintaining the shape already produced, or in shaping the hair and simultaneously fixing it.

The hair products for shaping and/or holding the hairstyle that are the most widespread on the cosmetics market are spray compositions, such as lacquers and sprays. They are essentially formed from a solution, which is usually alcoholic or aqueous, and of one or more materials, generally polymer resins, also known as fixing materials, whose function is to form welds between the hair, as a mixture with various cosmetic adjuvants. This composition constitutes the liquid phase of the hair product and is generally referred to as the fluid. It is applied to the hair, via an aerosol device, by means of a propellant.

Generally, the cosmetically acceptable medium is an alcoholic or aqueous-alcoholic medium. The propellant is a liquefied gas under reduced pressure or dissolved in a liquid phase. This propellant has the function of generating a pressure for spraying the liquid phase and for applying it to the hair in the form of a cloud of dispersed droplets.

Once applied to the hair, the liquid phase dries, thus enabling the formation of welds necessary for fixing the head of hair by the fixing materials.

The welds must be rigid enough to ensure that the hair is held in place. However, they must also be fragile enough for the user to be able, by combing or brushing the head of hair, to destroy them without damaging the scalp or the hair.

The aerosol sprays conventionally used generate a conical spray. This type of spray is well suited to the overall fixing of the head of hair and allows the hair to be held in place satisfactorily in the desired shape.

However, the use of these sprays is not optimal for localized application, especially at the root.

Specifically, it has been observed that the application of the fixing hair composition at the roots gives the hairstyle volume.

The Applicant has now discovered, surprisingly and advantageously, that the use of the aerosol device, equipped with a diffuser for generating a fan-shaped spray, makes it possible to obtain the desired effect.

In particular, such a use makes it possible, firstly, to facilitate the application and distribution of the hair composition at the roots of the head of hair, and thus gives the hairstyle volume.

One subject of the present invention is thus an aerosol device formed by:
- a container containing an aerosol composition comprising one or more propellants and, in an alcoholic or aqueous-alcoholic medium, one or more fixing polymers, and
- a means for dispensing the said aerosol composition comprising a diffuser equipped with a slit-shaped end orifice that produces a fan-shaped spray.

Another subject of the invention concerns a process for treating and especially for shaping keratin fibres, in particular the hair, comprising the use of this device.

Another subject of the invention consists of the use of the said device for shaping keratin fibres, such as the hair, and especially for giving the hairstyle volume.

Other subjects, characteristics, aspects and advantages of the invention will emerge even more clearly on reading the description and the examples that follow.

In the text hereinbelow, unless otherwise indicated, the limits of a range of values are included in that range, especially in the expressions "between" and "ranging from".

In the text hereinbelow, the term "at least one" is equivalent to "one or more".

The aerosol devices are generally formed from a container, also known as a reservoir, and a dispensing means.

The container is pressurized and comprises the composition to be dispensed. The container containing the pressurized composition may be opaque or transparent. It may be made of glass, a polymer or a metal, optionally coated with a protective varnish coat.

The container is equipped at its top end with a valve that seals the system. The valves that are suitable for the devices according to the invention are especially valves with an internal restriction orifice of between 0.3 and 3 mm, preferably between 1 and 2.5 mm and even more preferentially between 1.3 and 2 mm, and with a nozzle whose orifice is between 0.2 and 0.6 mm, preferably between 0.3 and 0.5 mm and even more preferentially between 0.35 and 0.45 mm in size.

They are in particular valves sold by the companies Precision, Coster, Seaquist and Lindal.

The device, conditioned with such a valve, ensures the sealing of the system, and also the dispensing of the product through the diffuser.

Onto this valve is fitted a dispensing means, on which the user can press to make the product come out.

This dispensing means is also known as a diffuser.

The diffuser according to the invention is equipped with a slit-shaped end orifice.

For the purposes of the present invention, the term "end orifice" means an orifice that opens to the exterior of the aerosol device. This orifice may be preceded in the diffuser by one or more other orifices of any shape.

This orifice may constitute one of the ends of an end piece fitted onto a standard diffuser via its other end, the arrival of the composition to be dispensed into the end piece taking place via an orifice, which may be of any shape.

For the purposes of the present invention, the term "slit" means an elongated shape comprising a ratio of its largest dimension to its smallest dimension, along perpendicular axes, preferably greater than or equal to 2, even more preferentially greater than or equal to 5.

Depending on the shape of the slit (rectangle, ellipse or diamond), the large and small dimensions may be referred to as the length and width, large and small axis, or large and small diagonal.

Preferably, the shape of the end orifice is rectangular.

This diffuser thus allows flat diffusion of the spray, forming a fan, and localized application of the composition at the root.

The aerosol composition according to the present invention comprises an aqueous-alcoholic or alcoholic medium optionally comprising at least one additional organic solvent.

The alcohol used in the composition according to the present invention is preferably a monohydroxylated alcohol chosen from C₁-C₄ lower alcohols, such as ethanol, isopropanol, tert-butanol or n-butanol.

Preferably, the alcohol used is ethanol.

The alcohol concentration in the aerosol composition (i.e. with one or more propellants) according to the present invention is between 0.1% and 95% by weight, preferably between 5% and 90% by weight, even more preferentially between 10% and 60% by weight, better still between 20% and 50% by weight and even better still between 20% and 40% by weight, relative to the total weight of the aerosol composition.

For the purposes of the present invention, the term "organic solvent" means an organic compound that is liquid at a temperature of 25°C and at atmospheric pressure. Preferably, the organic compound is polar.

Additional organic solvents that may be used in the composition according to the present invention include polyols such as propylene glycol, polyethylene glycols and polyol ethers, and mixtures thereof.

Preferably, the aerosol composition comprises ethanol. Preferably, ethanol is the only organic solvent of the aerosol composition, besides the propellant(s).

The concentration of additional organic solvents in the composition according to the present invention is between 0 and 30% and preferably between 5% and 20% by weight relative to the total weight of the aerosol composition.

The concentration of water in the compositions according to the present invention is between 0 and 50% by weight and preferably between 5% and 30% by weight relative to the total weight of the aerosol composition.

As indicated previously, the aerosol composition contained in the aerosol device according to the invention comprises one or more fixing polymers.

The term "fixing polymer" means any polymer that is capable of giving shape to a head of hair or of holding a head of hair in place in a given shape.

Preferably, the fixing polymer is chosen from anionic, amphoteric and nonionic fixing polymers.

The fixing polymers may be soluble in the aqueous-alcoholic or alcoholic medium or insoluble in this same medium and used in this case in the form of dispersions of solid or liquid particles of polymer, such as latices or pseudolatices.

The anionic fixing polymers generally used are polymers comprising groups derived from carboxylic acid, sulfonic acid or phosphoric acid and have a number-average molecular mass of between 500 and 5 000 000 approximately.

The carboxylic groups are provided by unsaturated mono- or dicarboxylic acid monomers such as those corresponding to formula (I): 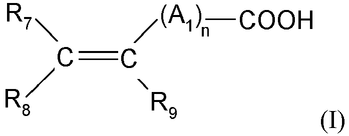 in which n is an integer from 0 to 10, A₁ denotes a methylene group optionally joined to the carbon atom of the unsaturated group or to the adjacent methylene group when n is greater than 1, via a heteroatom such as oxygen or sulfur, R₇ denotes a hydrogen atom or a phenyl or benzyl group, R₈ denotes a hydrogen atom, a lower alkyl or carboxyl group, R₉ denotes a hydrogen atom, a lower alkyl group, or a -CH₂-COOH, phenyl or benzyl group.

In the abovementioned formula (I), a lower alkyl group preferably denotes a group containing 1 to 4 carbon atoms and in particular methyl and ethyl groups.

The anionic fixing polymers containing carboxylic groups that are preferred according to the invention are:
A) copolymers of acrylic acid or methacrylic acid or salts thereof, and of acrylamide, sold in the form of their sodium salts under the names Reten 421, 423 or 425 by the company Hercules;
B) copolymers of acrylic or methacrylic acid with a monoethylenic monomer such as ethylene, styrene, vinyl esters and acrylic or methacrylic acid esters, optionally grafted onto a polyalkylene glycol such as polyethylene glycol and optionally crosslinked. Such polymers are described in particular in French patent 1 222 944 and German patent application 2 330 956, the copolymers of this type comprising an optionally N-alkylated and/or hydroxyalkylated acrylamide unit in their chain as described in particular in Luxembourg patent applications 75370 and 75371 or sold under the name Quadramer by American Cyanamid. Mention may also be made of copolymers of acrylic acid and of C₁-C₄ alkyl methacrylate and terpolymers of vinylpyrrolidone, of acrylic acid and of methacrylate of C₁-C₂₀ alkyl, for example lauryl methacrylate, such as the product sold by the company ISP under the name Acrylidone® LM and methacrylic acid/ethyl acrylate/tert-butyl acrylate terpolymers such as the product sold under the name Luvimer® 100 P by the company BASF.
   Mention may also be made of methacrylic acid/acrylic acid/ethyl acrylate/methyl methacrylate copolymers as an aqueous dispersion, sold under the name Amerhold® DR 25 by the company Amerchol;
C) crotonic acid copolymers, such as those comprising vinyl acetate or propionate units in their chain and optionally other monomers such as allylic esters or methallylic esters, vinyl ether or vinyl ester of a linear or branched saturated carboxylic acid with a long hydrocarbon-based chain, such as those containing at least 5 carbon atoms, it being possible for these polymers optionally to be grafted or crosslinked, or alternatively another vinyl, allylic or methallylic ester monomer of an α- or β-cyclic carboxylic acid. Such polymers are described, inter alia, in French patents 1 222 944, 1 580 545, 2 265 782, 2 265 781, 1 564 110 and 2 439 798. Commercial products that come under this category are the resins 28-29-30, 26-13-14 and 28-13-10 sold by the company National Starch;
D) copolymers of C₄-C₈ monounsaturated carboxylic acids or anhydrides chosen from:
   - copolymers comprising (i) one or more maleic, fumaric or itaconic acids or anhydrides and (ii) one or more monomers chosen from vinyl esters, vinyl ethers, vinyl halides, phenylvinyl derivatives, acrylic acid and its esters, the anhydride functions of these copolymers optionally being monoesterified or monoamidated. Such polymers are described, in particular, in US patents 2 047 398, 2 723 248 and 2 102 113, and GB patent 839 805. Commercial products are especially those sold under the names Gantrez® AN or ES by the company ISP;
   - copolymers comprising (i) one or more maleic, citraconic or itaconic anhydride units and (ii) one or more monomers chosen from allylic or methallylic esters optionally comprising one or more acrylamide, methacrylamide, α-olefin, acrylic or methacrylic ester, acrylic or methacrylic acid or vinylpyrrolidone groups in their chain,
   - the anhydride functions of these copolymers optionally being monoesterified or monoamidated.
   These polymers are described, for example, in French patents 2 350 384 and 2 357 241 by the Applicant;
E) polyacrylamides comprising carboxylate groups.
   The homopolymers and copolymers comprising sulfonic groups are polymers comprising vinylsulfonic, styrenesulfonic, naphthalenesulfonic or acrylamidoalkylsulfonic units.

These polymers may be chosen especially from:
- polyvinyl sulfonic acid salts with a molecular mass of between 1000 and 100 000 approximately, and also copolymers with an unsaturated comonomer such as acrylic or methacrylic acids and esters thereof, and also acrylamide or derivatives thereof, vinyl ethers and vinylpyrrolidone;
- polystyrenesulfonic acid salts such as the sodium salts, sold, for example, under the names Flexan® 500 and Flexan® 130 by National Starch, these polymers being described in patent FR 2 198 719;
- polyacrylamidesulfonic acid salts, such as those mentioned in patent US 4 128 631 and more particularly polyacrylamidoethylpropanesulfonic acid sold under the name Cosmedia Polymer HSP 1180 by Henkel.

As another anionic fixing polymer that can be used according to the invention, mention may be made of the branched block anionic polymer sold under the name Fixate G-100 by the company Noveon.

According to the invention, the anionic fixing polymers are preferably selected from acrylic acid copolymers such as the acrylic acid/ethyl acrylate/N-tert-butylacrylamide terpolymers sold in particular under the name Ultrahold® Strong by the company BASF, copolymers derived from crotonic acid, such as vinyl acetate/vinyl tert-butylbenzoate/crotonic acid terpolymers and the crotonic acid/vinyl acetate/vinyl neododecanoate terpolymers sold especially under the name Resin 28-29-30 by the company National Starch, polymers derived from maleic, fumaric or itaconic acids or anhydrides with vinyl esters, vinyl ethers, vinyl halides, phenylvinyl derivatives and acrylic acid and esters thereof, such as the methyl vinyl ether/monoesterified maleic anhydride copolymers sold, for example, under the name Gantrez® by the company ISP, the copolymers of methacrylic acid and of methyl methacrylate sold under the name Eudragit® L by the company Rohm Pharma, the copolymers of methacrylic acid and of ethyl acrylate sold under the name Luvimer® MAEX or MAE by the company BASF, the vinyl acetate/crotonic acid copolymers sold under the name Luviset CA 66 by the company BASF, the vinyl acetate/crotonic acid copolymers grafted with polyethylene glycol sold under the name Aristoflex® A by the company BASF, and the polymer sold under the name Fixate G-100 by the company Noveon.

Among the anionic fixing polymers mentioned above, it is more particularly preferred in the context of the present invention to use the methyl vinyl ether/monoesterified maleic anhydride copolymers sold under the name Gantrez® ES 425 by the company ISP, the acrylic acid/ethyl acrylate/N-tert-butylacrylamide terpolymers sold under the name Ultrahold® Strong by the company BASF, the copolymers of methacrylic acid and of methyl methacrylate sold under the name Eudragit® L by the company Rohm Pharma, the vinyl acetate/vinyl tert-butylbenzoate/crotonic acid terpolymers and the crotonic acid/vinyl acetate/vinyl neododecanoate terpolymers sold under the name Resin 28-29-30 by the company National Starch, the copolymers of methacrylic acid and of ethyl acrylate sold under the name Luvimer® MAEX or MAE by the company BASF, the butyl acrylate/acrylic acid/methacrylic acid branched block polymers and the vinylpyrrolidone/acrylic acid/lauryl methacrylate terpolymers sold under the name Acrylidone® LM by the company ISP and the polymer sold under the name Fixate G-100 by the company Noveon.

The amphoteric fixing polymers that can be used in accordance with the invention can be chosen from polymers comprising units B and C distributed randomly in the polymer chain, where B denotes a unit derived from a monomer comprising one or more basic nitrogen atoms and C denotes a unit derived from an acid monomer comprising one or more carboxylic or sulfonic groups, or alternatively B and C can denote groups derived from carboxybetaine or sulfobetaine zwitterionic monomers.

B and C can also denote a cationic polymer chain comprising primary, secondary, tertiary or quaternary amine groups, in which at least one of the amine groups bears a carboxylic or sulfonic group connected via a hydrocarbon group or alternatively B and C form part of a chain of a polymer containing an α,β-dicarboxylic ethylene unit in which one of the carboxylic groups has been made to react with a polyamine comprising one or more primary or secondary amine groups.

The amphoteric fixing polymers corresponding to the definition given above that are more particularly preferred are chosen from the following polymers:
(1) Copolymers having acidic vinyl units and basic vinyl units, such as those resulting from the copolymerization of a monomer derived from a vinyl compound bearing a carboxylic group such as, more particularly, acrylic acid, methacrylic acid, maleic acid, α-chloroacrylic acid, and a basic monomer derived from a substituted vinyl compound containing one or more basic atoms, such as, more particularly, dialkylaminoalkyl methacrylate and acrylate, dialkylaminoalkylmethacrylamides and acrylamides. Such compounds are described in patent US 3 836 537;
(2) Polymers comprising units derived from:
   a) at least one monomer chosen from acrylamides and methacrylamides substituted on the nitrogen atom with an alkyl group,
   b) at least one acidic comonomer containing one or more reactive carboxylic groups, and
   c) at least one basic comonomer such as esters containing primary, secondary, tertiary and quaternary amine substituents of acrylic and methacrylic acids and the product of quaternization of dimethylaminoethyl methacrylate with dimethyl or diethyl sulfate.
   The N-substituted acrylamides or methacrylamides that are more particularly preferred according to the invention are compounds in which the alkyl groups contain from 2 to 12 carbon atoms and more particularly N-ethylacrylamide, N-tert-butylacrylamide, N-tert-octylacrylamide, N-octylacrylamide, N-decylacrylamide, N-dodecylacrylamide and the corresponding methacrylamides.
   The acidic comonomers are chosen more particularly from acrylic acid, methacrylic acid, crotonic acid, itaconic acid, maleic acid and fumaric acid and alkyl monoesters, having 1 to 4 carbon atoms, of maleic or fumaric acids or anhydrides.
   The preferred basic comonomers are aminoethyl, butylaminoethyl, N,N'-dimethylaminoethyl and N-tert-butylaminoethyl methacrylates.
   Use is made particularly of the copolymers whose CTFA (4th edition, 1991) name is octylacrylamide/acrylates/butylaminoethyl methacrylate copolymer, such as the products sold under the name Amphomer® or Lovocryl® 47 by the company National Starch;
(3) Crosslinked and acylated polyaminoamides partially or totally deriving from polyaminoamides of general formula (II): 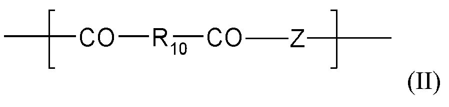 in which R₁₀ represents a divalent group derived from a saturated dicarboxylic acid, a mono- or dicarboxylic aliphatic acid containing an ethylenic double bond, an ester of a lower alkanol, containing 1 to 6 carbon atoms, of these acids, or a group derived from the addition of any one of said acids to a bis(primary) or bis(secondary) amine, and Z denotes a group derived from a bis(primary), mono- or bis(secondary) polyalkylene-polyamine and preferably represents:
   a) in proportions of from 60 mol% to 100 mol%, the group of formula (III): 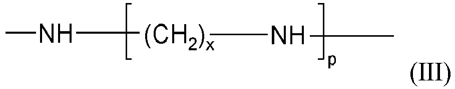 in which x = 2 and p = 2 or 3, or x = 3 and p = 2, this group being derived from diethylenetriamine, triethylenetetramine or dipropylenetriamine,
   b) in proportions of from 0 to 40 mol%, the group of formula (III) above in which x = 2 and p = 1 and which is derived from ethylenediamine, or the group derived from piperazine: 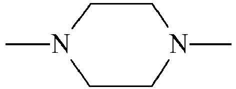
   c) in proportions of from 0 to 20 mol%, the -NH-(CH₂)₆-NH- group being derived from hexamethylenediamine, these polyamino amides being crosslinked by addition reaction of a difunctional crosslinking agent chosen from epihalohydrins, diepoxides, dianhydrides and bis-unsaturated derivatives, using from 0.025 to 0.35 mol of crosslinking agent per amine group of the polyamino amide and acylated by the action of acrylic acid, chloroacetic acid or an alkane sultone, or salts thereof.
   The saturated carboxylic acids are preferably selected from acids containing from 6 to 10 carbon atoms, such as adipic acid, 2,2,4-trimethyladipic acid and 2,4,4-trimethyladipic acid, terephthalic acid, acids containing an ethylenic double bond such as, for example, acrylic acid, methacrylic acid and itaconic acid.
   The alkane sultones used in the acylation are preferably propane sultone or butane sultone; the salts of the acylating agents are preferably the sodium or potassium salts;
(4) Polymers comprising zwitterionic units of formula (IV): 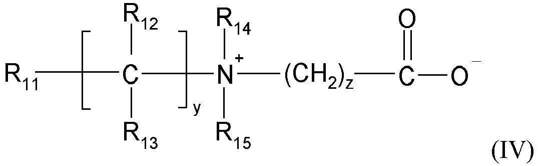 in which R₁₁ denotes a polymerizable unsaturated group such as an acrylate, methacrylate, acrylamide or methacrylamide group, y and z represent an integer from 1 to 3, R₁₂ and R₁₃ represent a hydrogen atom, a methyl, ethyl or propyl group, R₁₄ and R₁₅ represent a hydrogen atom or an alkyl group such that the sum of the carbon atoms in R₁₄ and R₁₅ does not exceed 10.
   The polymers comprising such units can also comprise units derived from non-zwitterionic monomers such as dimethyl- or diethylaminoethyl acrylate or methacrylate or alkyl acrylates or methacrylates, acrylamides or methacrylamides or vinyl acetate.
   By way of example, mention may be made of the copolymers of methyl methacrylate/methyl dimethylcarboxymethylammonioethyl methacrylate such as the product sold under the name Diaformer Z301 by the company Sandoz;
(5) Polymers derived from chitosan comprising monomer units corresponding to the following formulae: 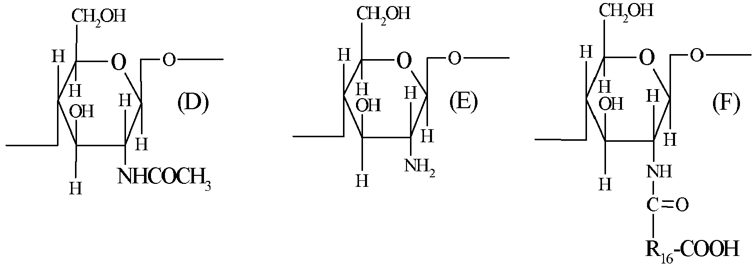 the unit (D) being present in proportions of between 0 and 30%, the unit (E) in proportions of between 5% and 50% and the unit (F) in proportions of between 30% and 90%, it being understood that, in this unit (F), R₁₆ represents a group of formula: 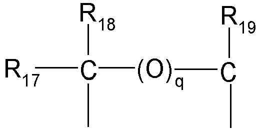
   in which, if q = 0, R₁₇, R₁₈ and R₁₉, which may be identical or different, each represent a hydrogen atom, a methyl, hydroxyl, acetoxy or amino residue, a monoalkylamine residue or a dialkylamine residue that are optionally interrupted by one or more nitrogen atoms and/or optionally substituted by one or more amine, hydroxyl, carboxyl, alkylthio or sulfonic groups, an alkylthio residue in which the alkyl group bears an amino residue, at least one of the groups R₁₇, R₁₈ and R₁₉ being, in this case, a hydrogen atom;
   or, if q = 1, R₁₇, R₁₈ and R₁₉ each represent a hydrogen atom, and also the salts formed by these compounds with bases or acids;
(6) Polymers corresponding to the general formula (V) are described, for example, in French patent 1 400 366: 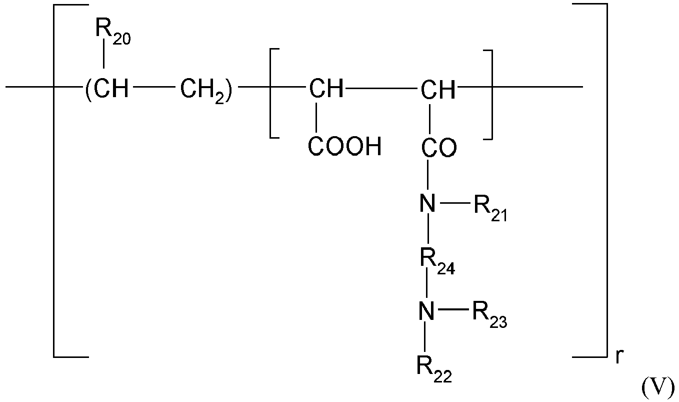 in which R₂₀ represents a hydrogen atom, a -O-CH₃, -O-CH₂-CH₃ or phenyl group, R₂₁ denotes a hydrogen atom or a lower alkyl group such as methyl or ethyl, R₂₂ denotes a hydrogen atom or a C₁-C₆ lower alkyl group such as methyl or ethyl, R₂₃ denotes a C₁-C₆ lower alkyl group such as methyl or ethyl or a group corresponding to the formula: -R₂₄-N(R₂₂)₂, R₂₄ representing a -CH₂-CH₂-, -CH₂-CH₂-CH₂- or -CH₂-CH(CH₃)-group, and R₂₂ having the abovementioned meanings;
(7) Polymers derived from the N-carboxyalkylation of chitosan, such as N-carboxymethylchitosan or N-carboxybutylchitosan sold under the name Evalsan by the company Jan Dekker;
(8) Amphoteric polymers of the type -D-X-D-X chosen from:
   a) Polymers obtained by the action of chloroacetic acid or sodium chloroacetate on compounds comprising at least one unit of formula (VI):

      -D-X-D-X-D- (VI)

      where D denotes a group 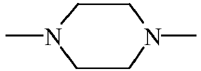 and X denotes the symbol E or E', E or E', which may be identical or different, denoting a divalent group that is an alkylene group with a straight or branched chain containing up to 7 carbon atoms in the main chain, which is unsubstituted or substituted by hydroxyl groups and which can comprise, in addition to the oxygen, nitrogen and sulfur atoms, 1 to 3 aromatic and/or heterocyclic rings; the oxygen, nitrogen and sulfur atoms being present in the form of ether, thioether, sulfoxide, sulfone, sulfonium, alkylamine or alkenylamine groups, hydroxyl, benzylamine, amine oxide, quaternary ammonium, amide, imide, alcohol, ester and/or urethane groups;
   b) Polymers of formula (VI'):

      -D-X-D-X- (VI')

      where D denotes a group 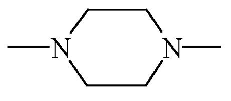 and X denotes the symbol E or E' and at least once E'; E having the meaning given above and E' is a divalent group that is an alkylene group with a straight or branched chain having up to 7 carbon atoms in the main chain, which is unsubstituted or substituted with one or more hydroxyl groups and containing one or more nitrogen atoms, the nitrogen atom being substituted with an alkyl chain that is optionally interrupted by an oxygen atom and necessarily comprising one or more carboxyl functions or one or more hydroxyl functions and betainized by reaction with chloroacetic acid or sodium chloroacetate;
(9) (C₁-C₅)alkyl vinyl ether/maleic anhydride copolymers partially modified by semiamidation with an N,N-dialkylaminoalkylamine such as N,N-dimethylaminopropylamine or by semiesterification with an N,N-dialkylaminoalkanol. These copolymers can also comprise other vinyl comonomers such as vinylcaprolactam.

Among the amphoteric fixing polymers mentioned above, the ones that are most particularly preferred according to the invention are those of family (3), such as the copolymers whose CTFA name is octylacrylamide/acrylates/butylaminoethyl methacrylate copolymer, such as the products sold under the names Amphomer®, Amphomer® LV 71 or Lovocryl® 47 by the company National Starch and those of family (4), such as the methyl methacrylate/methyl dimethylcarboxymethylammonioethyl methacrylate copolymers, sold, for example, under the name Diaformer Z301 by the company Sandoz.

The nonionic fixing polymers that may be used according to the present invention are chosen, for example, from:
- polyalkyloxazolines;
- vinyl acetate homopolymers;
- vinyl acetate copolymers, for instance copolymers of vinyl acetate and of acrylic ester; copolymers of vinyl acetate and of ethylene, or copolymers of vinyl acetate and of maleic ester, for example of dibutyl maleate;
- homopolymers and copolymers of acrylic esters, for instance copolymers of alkyl acrylates and of alkyl methacrylates, such as the products sold by the company Rohm & Haas under the names Primal® AC-261 K and Eudragit® NE 30 D, by the company BASF under the name 8845, or by the company Hoechst under the name Appretan® N9212;
- copolymers of acrylonitrile and of a nonionic monomer chosen, for example, from butadiene and alkyl (meth)acrylates, such as the products sold under the name CJ 0601 B by the company Rohm & Haas;
- styrene homopolymers;
- styrene copolymers, for instance copolymers of styrene and of alkyl (meth)acrylate, such as the products Mowilith® LDM 6911, Mowilith® DM 611 and Mowilith® LDM 6070 sold by the company Hoechst, and the products Rhodopas® SD 215 and Rhodopas® DS 910 sold by the company Rhône-Poulenc; copolymers of styrene, of alkyl methacrylate and of alkyl acrylate; copolymers of styrene and of butadiene; or copolymers of styrene, of butadiene and of vinylpyridine;
- polyamides;
- vinyllactam homopolymers such as vinylpyrrolidone homopolymers and such as the polyvinylcaprolactam sold under the name Luviskol® Plus by the company BASF; and
- vinyllactam copolymers such as a poly(vinylpyrrolidone/vinyllactam) copolymer sold under the trade name Luvitec® VPC 55K65W by the company BASF, poly(vinylpyrrolidone/vinyl acetate) copolymers, such as those sold under the name PVPVA® S630L by the company ISP, Luviskol® VA 73, VA 64, VA 55, VA 37 and VA 28 by the company BASF, and poly(vinylpyrrolidone/vinyl acetate/vinyl propionate) terpolymers, for instance the product sold under the name Luviskol® VAP 343 by the company BASF.

The alkyl groups of the nonionic polymers mentioned above preferably have from 1 to 6 carbon atoms.

According to the invention, it is also possible to use fixing polymers of grafted silicone type comprising a polysiloxane portion and a portion consisting of a non-silicone organic chain, one of the two portions constituting the main chain of the polymer, and the other being grafted onto said main chain.

These polymers are described, for example, in patent applications EP-A-412 704, EP-A-412 707, EP-A-640 105, WO 95/00578, EP-A-582 152 and WO 93/23009 and patents US 4 693 935, US 4 728 571 and US 4 972 037.

These polymers may be amphoteric, anionic or nonionic, and are preferably anionic or nonionic.

Such polymers are, for example, copolymers that can be obtained by free radical polymerization from the monomer mixture formed from:
a) 50% to 90% by weight of tert-butyl acrylate;
b) 0 to 40% by weight of acrylic acid;
c) 5% to 40% by weight of a silicone macromer of formula: 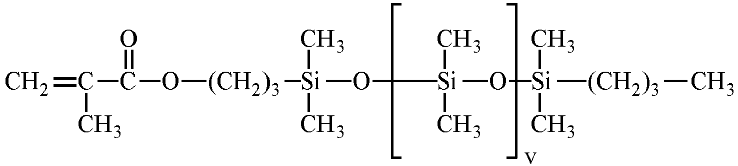 in which v is a number ranging from 5 to 700, the weight percentages being calculated relative to the total weight of the monomers.

Other examples of grafted silicone polymers are, in particular, polydimethylsiloxanes (PDMSs) onto which are grafted, via a thiopropylene-type connecting chain, mixed polymer units of the poly(meth)acrylic acid type and of the polyalkyl (meth)acrylate type and polydimethylsiloxanes (PDMSs) onto which are grafted, via a thiopropylene-type connecting chain, polymer units of the polyisobutyl (meth)acrylate type.

Another type of silicone fixing polymer that may be mentioned is the product Luviflex® Silk sold by the company BASF.

As fixing polymers it is also possible to use functionalized or non-functionalized, anionic, amphoteric or nonionic, silicone or non-silicone polyurethanes, or mixtures thereof.

The polyurethanes particularly targeted by the present invention are those disclosed in patent applications EP 0 751 162, EP 0 637 600, EP 0 648 485 and FR 2 743 297, of which the Applicant is the proprietor, and also patent applications EP 0 656 021 and WO 94/03510 from the company BASF and EP 0 619 111 from the company National Starch.

As polyurethanes that are particularly suitable for the present invention, mention may be made of the products sold under the names Luviset Pur® and Luviset® Si Pur by the company BASF.

Preferably, the fixing polymer(s) are present in proportions ranging from 0.1% to 20% by weight, preferably from 1% to 15% by weight and more preferentially from 3% to 10% by weight relative to the total weight of the aerosol composition.

According to one preferred embodiment, the solution or dispersion of one or more fixing polymers, in the alcoholic or aqueous-alcoholic medium, i.e. the liquid phase of the aerosol composition according to the invention, has a dynamic viscosity of greater than 3 cPs, preferably ranging from 3 to 20 cPs and even more preferentially from 4 to 10 cPs, before being placed in contact with the propellant, at a temperature of 25°C and at a shear rate of 1 s⁻¹.

This viscosity may be measured using a viscometer, for example a controlled-stress viscometer in cone-plate geometry, for instance a Haake RS1 viscometer from Thermo Electron.

The aerosol composition according to the invention comprises at least one propellant. This or these agents are chosen from dimethyl ether (DME), and volatile hydrocarbons such as n-butane, propane or isobutane, which are optionally chlorinated and/or fluorinated, and mixtures thereof. Among the fluorinated and/or chlorinated hydrocarbons, mention may be made of the compounds sold by the company DuPont de Nemours under Freon® and Dymel®, and in particular monofluorotrichloromethane, difluorodichloromethane, tetrafluorodichloroethane and 1,1-difluoroethane, sold especially under the trade name Dymel 152 A by the company DuPont de Nemours. Carbon dioxide, nitrous oxide, nitrogen or compressed air may also be used as propellant.

Dimethyl ether is preferably used.

Advantageously, the propellant(s) are present in a concentration ranging from 10% to 80% by weight, preferably from 20% to 75% by weight and even more preferentially from 30% to 70% by weight relative to the total weight of the aerosol composition.

The aerosol composition according to the invention may also comprise one or more thickeners.

For the purposes of the present invention, the term "thickener" means an agent which, when introduced at 1% by weight in an aqueous solution or an aqueous-alcoholic solution containing 30% ethanol, and at pH 7, makes it possible to achieve a dynamic viscosity of at least 100 cPs and preferably of at least 500 cPs, at 25°C and at a shear rate of 1 s⁻¹. This viscosity may be measured using a cone/plate viscometer as mentioned above.

These thickeners for increasing the viscosity of the composition contained in the device may be chosen from natural or synthetic, anionic, amphoteric, zwitterionic, nonionic or cationic, associative or non-associative polymeric thickeners.

Mention may be made, as polymeric thickeners, for example, of cellulose thickeners, such as hydroxyethylcellulose, hydroxypropylcellulose and carboxymethyl-cellulose, guar gum and derivatives thereof, such as hydroxypropyl guar, sold by the company Rhodia under the reference Jaguar HP 105, gums of microbial origin, such as xanthan gum and scleroglucan gum, synthetic polymeric thickening agents, such as crosslinked homopolymers of acrylic acid or of acrylamidopropanesulfonic acid, for instance Carbomer, or nonionic, anionic or amphoteric associative polymers, such as the polymers sold under the names Pemulen TR1 or TR2 by the company Goodrich, Salcare SC90 by the company Allied Colloids, Aculyn 22, 28, 33, 44 or 46 by the company Rohm & Haas, and Elfacos T210 and T212 by the company Akzo.

The aerosol composition contained in the device according to the invention may also contain one or more silicones, one or more non-silicone fatty substances, and one or more acidifying or basifying agents, and mixtures thereof.

The composition contained in the device according to the invention may also comprise one or more silicones.

The silicones that may be present in the composition according to the invention are in particular polyorganosiloxanes, which may be in the form of aqueous solutions, i.e. dissolved, or optionally in the form of dispersions or microdispersions, or of aqueous emulsions. The polyorganosiloxanes may also be in the form of oils, waxes, resins or gums.

Organopolysiloxanes are defined in greater detail in Walter Noll's Chemistry and Technology of Silicones, (1968), Academic Press.

The silicones may be volatile or non-volatile.

When they are volatile, the silicones are more particularly chosen from those with a boiling point of between 60°C and 260°C, and even more particularly from:
- cyclic silicones comprising from 3 to 7 and preferably 4 to 5 silicon atoms.

These are, for example, octamethylcyclotetrasiloxane sold especially under the name Volatile Silicone 7207 by the company Union Carbide or Silbione 70045 V 2 by the company Rhodia, decamethylcyclopentasiloxane sold under the name Volatile Silicone 7158 by the company Union Carbide, and Silbione 70045 V 5 by the company Rhodia, and mixtures thereof.

Mention may also be made of cyclocopolymers of the dimethylsiloxane/methylalkylsiloxane type, such as Volatile Silicone FZ 3109 sold by the company Union Carbide, of chemical structure:

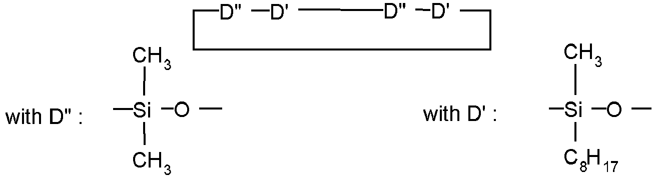

Mention may also be made of mixtures of cyclic silicones with organosilicon compounds, such as the mixture of octamethyl-cyclotetrasiloxane and tetrakis(trimethylsilyl)pentaerythritol (50/50) and the mixture of octamethylcyclotetrasiloxane and oxy-1,1'-bis(2,2,2',2',3,3'-hexatrimethylsilyloxy)neopentane;
- linear volatile silicones containing 2 to 9 silicon atoms and having a viscosity of less than or equal to 5×10⁻⁶ m²/s at 25°C. An example is decamethyltetrasiloxane sold in particular under the name SH 200 by the company Toray Silicone. Silicones belonging to this category are also described in the article published in Cosmetics and Toiletries, Vol. 91, Jan. 76, pp. 27-32 - Todd & Byers Volatile Silicone Fluids for Cosmetics*.*

Non-volatile silicones and more particularly polyalkylsiloxanes, polyarylsiloxanes, polyalkylarylsiloxanes, silicone gums and resins, and polyorganosiloxanes modified with organofunctional groups, and mixtures thereof, are preferably used.

These silicones are more particularly chosen from polyalkylsiloxanes, among which mention may be made mainly of polydimethylsiloxanes containing trimethylsilyl end groups (Dimethicone according to the CTFA name) having a viscosity of from 5×10⁻⁶ to 2.5 m²/s at 25°C and preferably 1×10⁻⁵ to 1 m²/s. The viscosity of the silicones is measured, for example, at 25°C according to standard ASTM 445 Appendix C.

Among these polyalkylsiloxanes, mention may be made, in a non-limiting manner, of the following commercial products:
- the Silbione oils of the 47 and 70 047 series or the Mirasil oils sold by the company Rhodia, for instance the oil 70 047 V 500 000,
- the oils of the Mirasil series sold by the company Rhodia,
- the oils of the 200 series from the company Dow Corning, such as, more particularly, DC200 with a viscosity of 60 000 cSt,
- the Viscasil oils from the company General Electric and certain oils of the SF series (SF 96, SF 18) from the company General Electric.

Mention may also be made of polydimethylsiloxanes containing dimethylsilanol end groups (Dimethiconol according to the CTFA name) such as the oils of the 48 series from the company Rhodia.

Mention may also be made of polydimethylsiloxanes containing aminoethyl, aminopropyl and α,ω-silanol groups.

In this category of polyalkylsiloxanes, mention may also be made of the products sold under the names Abil Wax 9800 and 9801 by the company Goldschmidt, which are poly(C₁-C₂₀)alkylsiloxanes.

The polyalkylarylsiloxanes are particularly chosen from linear and/or branched polydimethylmethylphenylsiloxanes and polydimethyldiphenylsiloxanes with a viscosity of from 1×10⁻⁵ to 5×10⁻² m²/s at 25°C.

Among these polyalkylarylsiloxanes, examples that may be mentioned include the products sold under the following names:
- Silbione oils of the 70 641 series from the company Rhodia,
- the oils of the Rhodorsil 70 633 and 763 series from the company Rhodia,
- the oil Dow Corning 556 Cosmetic Grade Fluid from the company Dow Corning,
- silicones of the PK series from the company Bayer, such as the product PK20,
- the silicones of the PN and PH series from the company Bayer, such as the products PN1000 and PH1000,
- certain oils of the SF series from the company General Electric, such as SF 1023, SF 1154, SF 1250 and SF 1265.

The silicone gums that may be present in the composition according to the invention are especially polydiorganosiloxanes having high number-average molecular masses of between 200 000 and 1 000 000, used alone or as a mixture in a solvent. This solvent can be chosen from volatile silicones, polydimethylsiloxane (PDMS) oils, polyphenylmethylsiloxane (PPMS) oils, isoparaffins, polyisobutylenes, methylene chloride, pentane, dodecane and tridecane, or mixtures thereof.

Mention may be made more particularly of the following products:
- polydimethylsiloxane gums,
- polydimethylsiloxane/methylvinylsiloxane gums,
- polydimethylsiloxane/diphenylsiloxane gums,
- polydimethylsiloxane/phenylmethylsiloxane gums,
- polydimethylsiloxane/diphenylsiloxane/methylvinylsiloxane gums.

Products that may be used more particularly are the following mixtures:
- mixtures formed from a polydimethylsiloxane hydroxylated at the end of the chain (known as dimethiconol according to the nomenclature of the CTFA dictionary) and from a cyclic polydimethylsiloxane (known as cyclomethicone according to the nomenclature of the CTFA dictionary), such as the product Q2 1401 sold by the company Dow Corning,
- mixtures formed from a polydimethylsiloxane gum with a cyclic silicone, such as the product SF 1214 Silicone Fluid from the company General Electric, this product being an SF 30 gum corresponding to a dimethicone, having a number-average molecular weight of 500 000, dissolved in the oil SF 1202 Silicone Fluid corresponding to decamethylcyclopentasiloxane,
- mixtures of two PDMSs with different viscosities, and more particularly of a PDMS gum and a PDMS oil, such as the product SF 1236 from the company General Electric. The product SF 1236 is the mixture of a gum SE 30 defined above, with a viscosity of 20 m²/s and of an oil SF 96 with a viscosity of 5×10⁻⁶ m²/s. This product preferably comprises 15% of gum SE 30 and 85% of an oil SF 96.

The organopolysiloxane resins that may be present in the composition according to the invention are crosslinked siloxane systems containing the following units: R₂SiO₂/₂, R₃SiO₁/₂, RSiO₃/₂ and SiO₄/₂ in which R represents a hydrocarbon group containing 1 to 16 carbon atoms or a phenyl group. Among these products, those that are particularly preferred are the ones in which R denotes a C₁-C₄ lower alkyl radical, more particularly methyl, or a phenyl radical.

Among these resins, mention may be made of the product sold under the name Dow Corning 593 or those sold under the names Silicone Fluid SS 4230 and SS 4267 by the company General Electric, which are silicones of dimethyl/trimethylsiloxane structure.

Mention may also be made of the trimethyl siloxysilicate type resins sold in particular under the names X22-4914, X21-5034 and X21-5037 by the company Shin-Etsu.

The organomodified silicones that may be present in the composition according to the invention are silicones as defined above and comprising in their structure one or more organofunctional groups attached via a hydrocarbon-based radical.

Among the organomodified silicones, mention may be made of polyorganosiloxanes comprising:
- polyethyleneoxy and/or polypropyleneoxy groups optionally comprising C₆-C₂₄ alkyl groups, such as the products known as dimethicone copolyol sold by the company Dow Corning under the name DC 1248 or the oils Silwet L 722, L 7500, L 77 and L 711 by the company Union Carbide, and the (C₁₂)alkylmethicone copolyol sold by the company Dow Corning under the name Q2 5200,
- thiol groups, such as the products sold under the names GP 72 A and GP 71 from the company Genesee,
- alkoxylated groups, such as the product sold under the name Silicone Copolymer F-755 by SWS Silicones and Abil Wax 2428, 2434 and 2440 by the company Goldschmidt,
- hydroxylated groups, such as the polyorganosiloxanes containing a hydroxyalkyl function, described in French patent application FR-A-85 16334;
- acyloxyalkyl groups, for instance the polyorganosiloxanes described in patent US-A-4 957 732,
- anionic groups of the carboxylic acid type, for instance in the products described in patent EP 186 507 from the company Chisso Corporation, or of the alkylcarboxylic type, such as those present in the product X-22-3701E from the company Shin-Etsu; 2-hydroxyalkyl sulfonate; 2-hydroxyalkyl thiosulfate such as the products sold by the company Goldschmidt under the names Abil S201 and Abil S255,
- hydroxyacylamino groups, for instance the polyorganosiloxanes described in patent application EP 342 834. Mention may be made, for example, of the product Q2-8413 from the company Dow Corning.

Among the organomodified silicones, mention may also be made of amino silicones.

For the purposes of the present invention, the term "amino silicone" means any silicone comprising at least one primary, secondary or tertiary amine function or a quaternary ammonium group.

The amino silicones that may be used in the cosmetic composition according to the present invention are chosen from:
(a) the compounds corresponding to formula (VII) below:

   (R¹)ₐ(T)₃₋ₐ-Si[OSi(T)₂]ₙ-[OSi(T)_{b}(R¹)_{2-b}]ₘ-OSi(T)₃₋ₐ-(R¹)ₐ (VII)

   in which:
   T is a hydrogen atom or a phenyl, hydroxyl (-OH) or C₁-C₈ alkyl radical, and preferably methyl, or a C₁-C₈ alkoxy radical, preferably methoxy,
   a denotes the number 0 or an integer from 1 to 3, and preferably 0,
   b denotes 0 or 1, and in particular 1,
   m and n are numbers such that the sum (n + m) can range especially from 1 to 2000 and in particular from 50 to 150, it being possible for n to denote a number from 0 to 1999 and in particular from 49 to 149, and for m to denote a number from 1 to 2000 and in particular from 1 to 10,
   R¹ is a monovalent radical of formula -C_{q}H_{2q}L in which q is a number from 2 to 8 and L is an optionally quaternized amino group chosen from the following groups:
      -N(R²)-CH₂-CH₂-N(R²)₂,
      -N(R²)₂,
      -N⁺(R²)₃Q⁻,
      -N⁺(R²)(H)₂Q⁻,
      -N⁺(R²)₂HQ⁻,
      -N(R²)-CH₂-CH₂-N⁺(R²)(H)₂Q⁻,
   in which R² may denote a hydrogen atom, a phenyl, a benzyl or a saturated monovalent hydrocarbon-based group, for example a C₁-C₂₀ alkyl group, and Q⁻ represents a halide ion, for instance fluoride, chloride, bromide or iodide.

In particular, the amino silicones corresponding to the definition of formula (VII) are chosen from the compounds corresponding to formula (VIII) below: 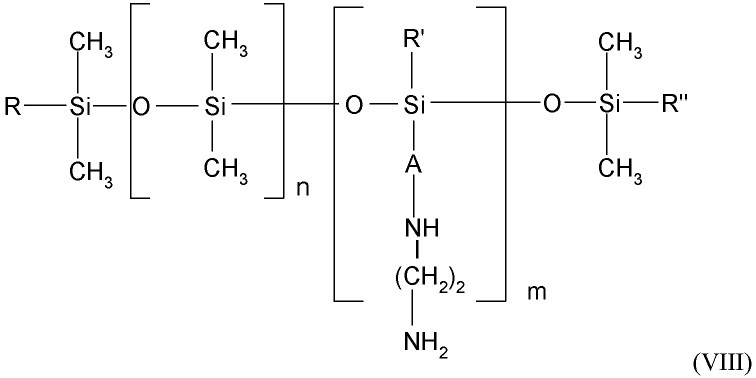 in which R, R' and R", which may be identical or different, denote a C₁-C₄ alkyl radical, preferably CH₃, a C₁-C₄ alkoxy radical, preferably methoxy, or OH, A represents a linear or branched C₃-C₈ and preferably C₃-C₆ alkylene radical, m and n are integers dependent on the molecular weight and whose sum is between 1 and 2000.

According to a first possibility, R, R' and R", which may be identical or different, each represent a C₁-C₄ alkyl or hydroxyl radical, A represents a C₃ alkylene radical and m and n are such that the weight-average molecular mass of the compound is between 5000 and 500 000 approximately. Compounds of this type are referred to in the CTFA dictionary as "amodimethicones".

According to a second possibility, R, R' and R", which may be identical or different, each represent a C₁-C₄ alkoxy or hydroxyl radical, at least one of the radicals R or R" is an alkoxy radical and A represents a C₃ alkylene radical. The hydroxyl/alkoxy mole ratio is preferably between 0.2/1 and 0.4/1 and advantageously equal to 0.3/1. Moreover, m and n are such that the weight-average molecular mass of the compound is between 2000 and 10⁶. More particularly, n is between 0 and 999 and m is between 1 and 1000, the sum of n and m being between 1 and 1000.

In this category of compounds, mention may be made, inter alia, of the product Belsil® ADM 652 sold by the company Wacker.

According to a third possibility, R and R", which are different, each represent a C₁-C₄ alkoxy or hydroxyl radical, at least one of the radicals R or R" being an alkoxy radical, R' representing a methyl group and A representing a C₃ alkylene radical. The hydroxyl/alkoxy mole ratio is preferably between 1/0.8 and 1/1.1 and advantageously is equal to 1/0.95. Moreover, m and n are such that the weight-average molecular weight of the compound is between 2000 and 200 000. More particularly, n is between 0 and 999 and m is between 1 and 1000, the sum of n and m being between 1 and 1000.

More particularly, mention may be made of the product Fluid WR® 1300 sold by the company Wacker.

Note that the molecular mass of these silicones is determined by gel permeation chromatography (ambient temperature, polystyrene standard, µ styragem columns, eluent THF, flow rate of 1 mm/minute, 200 µl of a solution containing 0.5% by weight of silicone in THF are injected, and detection is performed by refractometry and UV-metry).

A product corresponding to the definition of formula (VII) is in particular the polymer known in the CTFA dictionary as "trimethylsilyl amodimethicone", corresponding to formula (IX) below: 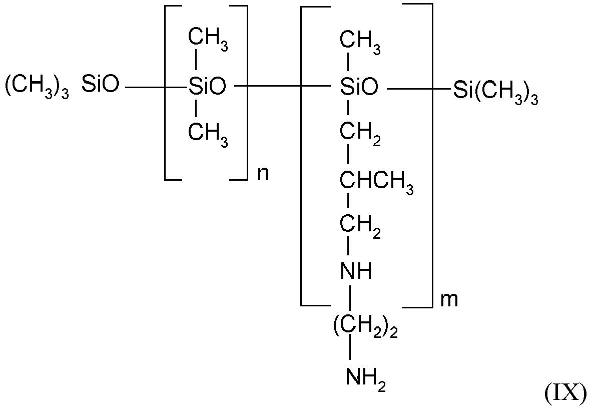 in which n and m have the meanings given above in accordance with formula (VII).

Such compounds are described, for example, in patent EP 95238. A compound of formula (IX) is sold, for example, under the name Q2-8220 by the company OSI.
(b) the compounds corresponding to formula (X) below: 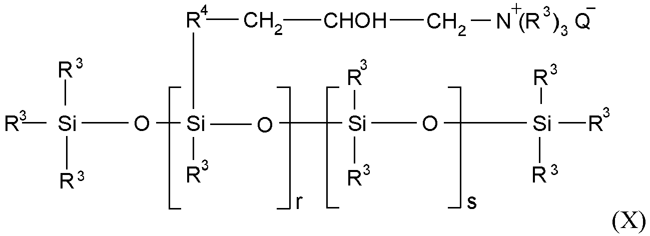 in which:
   R³ represents a C₁-C₁₈ monovalent hydrocarbon-based radical, and in particular a C₁-C₁₈ alkyl or C₂-C₁₈ alkenyl radical, for example methyl;
   R⁴ represents a divalent hydrocarbon-based radical, especially a C₁-C₁₈ alkylene radical or a divalent C₁-C₁₈, and for example C₁-C₈, alkylenoxy radical;
   Q⁻ is a halide ion, in particular chloride;
   r represents a mean statistical value from 2 to 20 and in particular from 2 to 8;
   s represents a mean statistical value from 20 to 200 and in particular from 20 to 50.
   Such compounds are described more particularly in patent US 4 185 087.
   A compound falling within this class is the product sold by the company Union Carbide under the name Ucar Silicone ALE 56.
(c) the quaternary ammonium silicones of formula (XI): 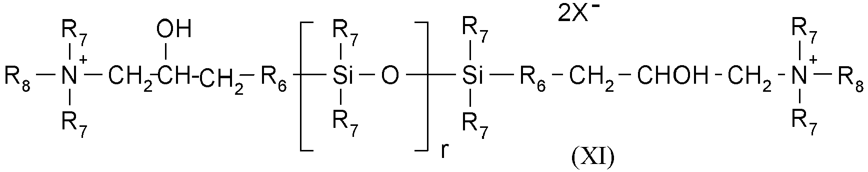 in which:
   R₇, which may be identical or different, represent a monovalent hydrocarbon-based radical containing from 1 to 18 carbon atoms, and in particular a C₁-C₁₈ alkyl radical, a C₂-C₁₈ alkenyl radical or a ring comprising 5 or 6 carbon atoms, for example methyl;
   R₆ represents a divalent hydrocarbon-based radical, especially a C₁-C₁₈ alkylene radical or a divalent C₁-C₁₈, and for example C₁-C₈, alkylenoxy radical linked to the Si via an Si-C bond;
   R₈, which may be identical or different, each represent a hydrogen atom, a monovalent hydrocarbon-based radical containing from 1 to 18 carbon atoms, and in particular a C₁-C₁₈ alkyl radical, a C₂-C₁₈ alkenyl radical or a radical -R₆-NHCOR₇;
   X⁻ is an anion such as a halide ion, especially chloride, or an organic acid salt (acetate, etc.);
   r represents a mean statistical value from 2 to 200 and in particular from 5 to 100.
   These silicones are described, for example, in patent application EP-A-0 530 974.
(d) the amino silicones of formula (XII): 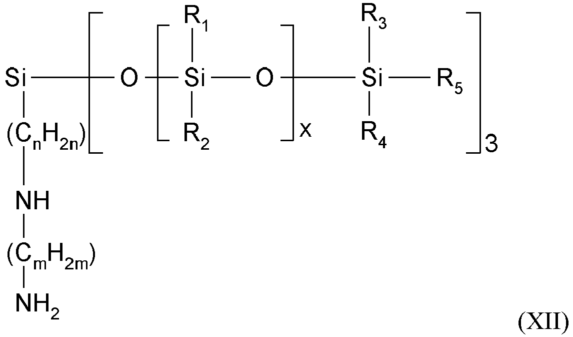 in which:
   - R₁, R₂, R₃ and R₄, which may be identical or different, denote a C₁-C₄ alkyl group or a phenyl group,
   - R₅ denotes a C₁-C₄ alkyl radical or a hydroxyl group,
   - n is an integer ranging from 1 to 5,
   - m is an integer ranging from 1 to 5, and
   - x is chosen such that the amine number is between 0.01 and 1 meq/g.

The silicones that are particularly preferred are polydimethylsiloxanes (INCI name: dimethicone) and amino silicones.

When these compounds are used, one particularly advantageous embodiment involves their combined use with cationic and/or nonionic surfactants.

By way of example, use may be made of the product sold under the name Cationic Emulsion DC 939 by the company Dow Corning, which comprises, besides amodimethicone, a cationic surfactant which is trimethylcetylammonium chloride and a nonionic surfactant of formula: C₁₃H₂₇-(OC₂H₄)₁₂-OH, known under the CTFA name Trideceth-12.

Another commercial product that may be used according to the invention is the product sold under the name Dow Corning Q2 7224 by the company Dow Corning, comprising, in combination, the trimethylsilyl amodimethicone of formula (IX) described above, a nonionic surfactant of formula: C₈H₁₇-C₆H₄-(OCH₂CH₂)₄₀-OH, known under the CTFA name Octoxynol-40, a second nonionic surfactant of formula: C₁₂H₂₅-(OCH₂-CH₂)₆-OH, known under the CTFA name Isolaureth-6, and propylene glycol.

The composition contained in the device according to the invention may also comprise one or more non-silicone fatty substances.

The term "fatty substance" means an organic compound that is insoluble in water at room temperature (25°C) and at atmospheric pressure (760 mmHg, i.e. 1.013×10⁵ Pa), i.e. with a solubility of less than 5%, preferably of less than 1% and even more preferably of less than 0.1%. The non-silicone fatty substances generally have in their structure a hydrocarbon-based chain comprising at least 6 carbon atoms and not comprising any siloxane groups. In addition, the fatty substances are generally soluble in organic solvents under the same temperature and pressure conditions, for instance chloroform, ethanol, benzene, liquid petroleum jelly or decamethylcyclopentasiloxane.

The term "non-silicone fatty substance" means a fatty substance whose structure does not comprise any silicon atoms.

The fatty substances that may be used in the composition according to the invention are generally not oxyalkylenated and preferably do not contain any carboxylic acid COOH functions.

Preferably, the non-silicone fatty substances of the invention are chosen from hydrocarbons, fatty alcohols, fatty esters, silicones and fatty ethers, and mixtures thereof.

Even more preferentially, they are chosen from hydrocarbons, fatty alcohols and fatty esters, and mixtures thereof.

They may be liquid or non-liquid at room temperature and at atmospheric pressure.

The liquid fatty substances of the invention preferably have a viscosity of less than or equal to 2000 cPs, better still less than or equal to 1000 cPs and even better still less than or equal to 100 cPs at a temperature of 25°C and at a shear rate of 1 s⁻¹. This viscosity may be measured using a viscometer with cone/plate geometry as mentioned above.

The term "liquid hydrocarbon" means a hydrocarbon composed solely of carbon and hydrogen atoms, which is liquid at room temperature (25°C) and at atmospheric pressure (760 mmHg, i.e. 1.013×10⁵ Pa).

More particularly, the liquid hydrocarbons are chosen from:
- linear or branched, optionally cyclic, C₆-C₁₆ lower alkanes. Examples that may be mentioned include hexane, undecane, dodecane, tridecane, and isoparaffins, for instance isohexadecane, isododecane and isodecane;
- linear or branched hydrocarbons of mineral, animal or synthetic origin with more than 16 carbon atoms, such as volatile or non-volatile liquid paraffins and derivatives thereof, petroleum jelly, liquid petroleum jelly, polydecenes, hydrogenated polyisobutene and squalane.

In one preferred variant, the liquid hydrocarbon(s) are chosen from volatile or non-volatile liquid paraffins, and derivatives thereof, and liquid petroleum jelly.

The term "liquid fatty alcohol" means a non-glycerolated and non-oxyalkylenated fatty alcohol, which is liquid at room temperature (25°C) and at atmospheric pressure (760 mmHg, i.e. 1.013×10⁵ Pa).

Preferably, the liquid fatty alcohols of the invention comprise from 8 to 30 carbon atoms.

The liquid fatty alcohols of the invention may be saturated or unsaturated.

The saturated liquid fatty alcohols are preferably branched. They may optionally comprise in their structure at least one aromatic or non-aromatic ring. They are preferably acyclic.

More particularly, the liquid saturated fatty alcohols of the invention are chosen from octyldodecanol, isostearyl alcohol and 2-hexyldecanol.

Octyldodecanol is most particularly preferred.

The unsaturated liquid fatty alcohols contain in their structure at least one double or triple bond, and preferably one or more double bonds. When several double bonds are present, there are preferably 2 or 3 of them, and they may be conjugated or unconjugated.

These unsaturated fatty alcohols may be linear or branched.

They may optionally comprise in their structure at least one aromatic or non-aromatic ring. They are preferably acyclic.

More particularly, the unsaturated liquid fatty alcohols of the invention are chosen from oleyl alcohol, linoleyl alcohol, linolenyl alcohol and undecylenyl alcohol.

Oleyl alcohol is most particularly preferred.

The term "liquid fatty esters" means an ester derived from a fatty acid and/or from a fatty alcohol that is liquid at room temperature (25°C) and at atmospheric pressure (760 mmHg, i.e. 1.013 × 10⁵ Pa).

The esters are preferably liquid esters of saturated or unsaturated, linear or branched C₁-C₂₆ aliphatic monoacids or polyacids and of saturated or unsaturated, linear or branched C₁-C₂₆ aliphatic monoalcohols or polyalcohols, the total number of carbon atoms of the esters being greater than or equal to 10.

Preferably, for the esters of monoalcohols, at least one from among the alcohol and the acid from which the esters of the invention are derived is branched.

Among the monoesters of monoacids and of monoalcohols, mention may be made of ethyl palmitate, isopropyl palmitate, alkyl myristates such as isopropyl myristate or ethyl myristate, isocetyl stearate, 2-ethylhexyl isononanoate, isodecyl neopentanoate and isostearyl neopentanoate.

Esters of C₄-C₂₂ dicarboxylic or tricarboxylic acids and of C₁-C₂₂ alcohols and esters of monocarboxylic, dicarboxylic or tricarboxylic acids and of C₄-C₂₆ dihydroxy, trihydroxy, tetrahydroxy or pentahydroxy alcohols may also be used.

Mention may be made especially of: diethyl sebacate; diisopropyl sebacate; bis(2-ethylhexyl) sebacate; diisopropyl adipate; di-n-propyl adipate; dioctyl adipate; bis(2-ethylhexyl) adipate; diisostearyl adipate; bis(2-ethylhexyl) maleate; triisopropyl citrate; triisocetyl citrate; triisostearyl citrate; glyceryl trilactate; glyceryl trioctanoate; trioctyldodecyl citrate; trioleyl citrate; neopentyl glycol diheptanoate; diethylene glycol diisononanoate.

The composition may also comprise, as liquid fatty ester, sugar esters and diesters of C₆-C₃₀ and preferably C₁₂-C₂₂ fatty acids. It is recalled that the term "sugar" means oxygen-bearing hydrocarbon-based compounds containing several alcohol functions, with or without aldehyde or ketone functions, and which comprise at least 4 carbon atoms. These sugars may be monosaccharides, oligosaccharides or polysaccharides.

Examples of suitable sugars that may be mentioned include saccharose, glucose, galactose, ribose, fucose, maltose, fructose, mannose, arabinose, xylose and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated C₆-C₃₀ and preferably C₁₂-C₂₂ fatty acids. If they are unsaturated, these compounds may comprise one to three conjugated or unconjugated carbon-carbon double bonds.

The esters according to this variant may also be chosen from mono-, di-, tri- and tetraesters, and polyesters, and mixtures thereof.

These esters may be chosen, for example, from oleates, laurates, palmitates, myristates, behenates, cocoates, stearates, linoleates, linolenates, caprates and arachidonates, or mixtures thereof, such as, in particular, oleopalmitate, oleostearate or palmitostearate mixed esters.

More particularly, use is made of monoesters and diesters and in particular of sucrose, glucose or methylglucose mono- or dioleates, stearates, behenates, oleopalmitates, linoleates, linolenates or oleostearates.

An example that may be mentioned is the product sold under the name Glucate® DO by the company Amerchol, which is a methylglucose dioleate.

Finally, use may also be made of natural or synthetic glycerol esters of mono-, di- or triacids.

Among these, mention may be made of plant oils.

As oils of plant origin or synthetic triglycerides that may be used in the composition of the invention as liquid fatty esters, examples that may be mentioned include:
- triglyceride oils of vegetable or synthetic origin, such as liquid triglycerides of fatty acids comprising from 6 to 30 carbon atoms, such as triglycerides of heptanoic or octanoic acids, or also, for example, sunflower, maize, soybean, cucumber, grape seed, sesame, hazelnut, apricot, macadamia, arara, castor or avocado oils, triglycerides of caprylic/capric acids, such as those sold by the company Stearineries Dubois or those sold under the names Miglyol® 810, 812 and 818 by the company Dynamit Nobel, jojoba oil or shea butter oil.

Liquid fatty esters derived from monoalcohols will preferably be used as esters according to the invention.

Isopropyl myristate and isopropyl palmitate are particularly preferred.

The liquid fatty ethers are chosen from liquid dialkyl ethers such as dicaprylyl ether.

The fatty substance(s) in the composition according to the invention may also be liquid or non-liquid at room temperature (25°C) and at atmospheric pressure (760 mmHg, i.e. 1.013×10⁵ Pa).

The term "non-liquid" preferably means a solid compound or a compound that has a viscosity of greater than 2000 cPs at room temperature (25°C) and at a shear rate of 1 s⁻¹. This viscosity may be measured using a viscometer with cone/plate geometry as mentioned above.

More particularly, the non-liquid fatty substances are chosen from fatty alcohols, fatty acid and/or fatty alcohol esters, non-silicone waxes and fatty ethers, which are non-liquid and preferably solid.

The non-liquid fatty alcohols that are suitable for use in the invention are more particularly chosen from saturated or unsaturated, linear or branched alcohols comprising from 8 to 30 carbon atoms. Mention may be made, for example, of cetyl alcohol, stearyl alcohol and a mixture thereof (cetylstearyl alcohol).

As regards the non-liquid esters of fatty acids and/or of fatty alcohols, mention may be made especially of solid esters derived from C₉-C₂₆ fatty acids and from C₉-C₂₆ fatty alcohols.

Among these esters, mention may be made of octyldodecyl behenate, isocetyl behenate, cetyl lactate, stearyl octanoate, octyl octanoate, cetyl octanoate, decyl oleate, myristyl stearate, octyl palmitate, octyl pelargonate, octyl stearate, alkyl myristates such as cetyl myristate, myristyl myristate and stearyl myristate, and hexyl stearate.

Still within the context of this variant, esters of C₄-C₂₂ dicarboxylic or tricarboxylic acids and of C₁-C₂₂ alcohols and esters of monocarboxylic, dicarboxylic or tricarboxylic acids and of C₂-C₂₆ dihydroxy, trihydroxy, tetrahydroxy or pentahydroxy alcohols may also be used.

Mention may be made especially of diethyl sebacate, diisopropyl sebacate, diisopropyl adipate, di-n-propyl adipate, dioctyl adipate and dioctyl maleate.

Among all the esters mentioned above, it is preferred to use myristyl, cetyl or stearyl palmitates, and alkyl myristates such as cetyl myristate, stearyl myristate and myristyl myristate.

The non-silicone wax(es) are chosen especially from carnauba wax, candelilla wax, esparto wax, paraffin wax, ozokerite, plant waxes such as olive tree wax, rice wax, hydrogenated jojoba wax or absolute flower waxes such as the blackcurrant blossom essential wax sold by the company Bertin (France), and animal waxes such as beeswaxes or modified beeswaxes (cerabellina).

Other waxes or waxy starting materials that may be used according to the invention are especially marine waxes such as those sold by the company Sophim under the reference M82, and waxes of polyethylene or of polyolefins in general.

The non-liquid fatty ethers are chosen from dialkyl ethers and especially dicetyl ether and distearyl ether, alone or as a mixture.

When the composition comprises solid compounds, the liquid phase is then in the form of a dispersion.

Preferably, the non-silicone fatty substance(s) are liquid at room temperature (25°C) and at atmospheric pressure (760 mmHg, i.e. 1.013 × 10⁵ Pa) and are chosen from mineral oils, plant oils such as olive oil, castor oil, rapeseed oil, wheatgerm oil, sweet almond oil, avocado oil, macadamia oil, apricot oil, safflower oil, candlenut oil, camellina oil, tamanu oil, lemon oil, alkanes and more particularly C₅-C₁₀ alkanes, C₁₀-C₃₀ liquid fatty esters such as octyldodecanol and oleyl alcohol, and more particularly liquid esters of C₈-C₂₀ fatty acids and of C₁-C₈ alcohols such as isopropyl myristate, liquid esters of C₉-C₃₀ fatty alcohols such as C₁₀-C₃₀ fatty alkyl benzoates, isononyl isononanoate, isostearyl malate and tridecyl trimellitate, and mixtures thereof, and liquid esters of polyols such as pentaerythrityl tetraisostearate.

If it contains water, the liquid phase contained in the device according to the invention generally has a pH ranging from 2 to 9, in particular from 3 to 8 and better still from 6 to 8. It may be adjusted to the desired value by means of acidifying or basifying agents usually used in cosmetics for this type of application, or alternatively using standard buffer systems.

Among the acidifying agents that may be mentioned, for example, are mineral or organic acids, for instance hydrochloric acid, orthophosphoric acid or sulfuric acid, carboxylic acids, for instance acetic acid, tartaric acid, citric acid and lactic acid, and sulfonic acids.

Among the basifying agents, examples that may be mentioned include aqueous ammonia, alkali metal carbonates, alkanolamines, such as mono-, di- and triethanolamines and 2-amino-2-methyl-1-propanol and derivatives thereof, sodium hydroxide, potassium hydroxide and the compounds having the following formula: 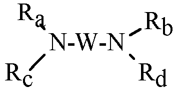 in which W is a propylene residue optionally substituted with a hydroxyl group or a C₁-C₄ alkyl radical; and Rₐ, R_{b}, R_{c} and R_{d}, which may be identical or different, represent a hydrogen atom or a C₁-C₄ alkyl or C₁-C₄ hydroxyalkyl radical.

Preferably, the pH modifiers may be chosen from alkaline agents, such as aqueous ammonia, monoethanolamine, diethanolamine, triethanolamine, 2-amino-2-methyl-1-propanol, 1,3-propanediamine or an alkali metal hydroxide, or from acidifying agents, such as phosphoric acid or hydrochloric acid.

The aerosol compositions contained in the device according to the invention may also contain at least one adjuvant chosen from nonionic, anionic, cationic, amphoteric and zwitterionic surfactants, ceramides and pseudoceramides, vitamins and provitamins including panthenol, water-soluble and liposoluble, silicone or non-silicone sunscreens, mineral and organic, coloured or uncoloured pigments, permanent or temporary dyes, nacreous agents and opacifiers, sequestrants, plasticizers, solubilizers, antioxidants, hydroxy acids, penetrants, fragrances, fragrance solubilizers (peptizers), preserving agents, anticorrosion agents and treating active agents.

A person skilled in the art will take care to select the optional additives and the amounts thereof so that they do not harm the properties of the compositions of the present invention.

These additives may be present in the composition according to the invention in an amount ranging from 0 to 20% by weight relative to the total weight of the composition.

Another subject of the invention concerns a process for treating, and especially for shaping, keratin fibres, in particular the hair, comprising the use of this device.

In particular, this process includes a step of applying the aerosol composition according to the invention to the said fibres using the device according to the invention.

For certain heads of hair, the shaping process may comprise a step enabling the user to expose the inaccessible roots of his hair.

The invention also relates to the use of a device comprising a diffuser, equipped with a slit-shaped orifice for producing a fan-shaped spray, as defined above, for the shaping of keratin fibres, such as the hair, and especially for giving the hairstyle volume.

The examples that follow illustrate the present invention, and should not in any way be considered as limiting the invention.

### EXAMPLES

In the following examples, all the amounts are shown as mass percentage of starting material in unmodified form, relative to the total weight of the composition.

### Preparation of the devices:

A device according to the invention: device A, and a comparative device: device B, not comprising a slit diffuser, are compared with each other.

Device A according to the invention and device B outside the invention were filled with the same composition, given in the table below.

| | |
|---|---|
| Vinyl acetate/vinyl tert-butylbenzoate/crotonic acid terpolymer (1) | 6% |
| 2-Amino-2-methyl-1-propanol | 0.6% (qs 100% neutralization) |
| Polyvinylpyrrolidone PVP K 90 (2) | 0.1% |
| Ethanol | 33.3% |
| Dimethyl ether | 60% |

| | |
|---|---|
| (1) Mexomer PW sold by the company Chimex (2) PVP K 90 sold by the company ISP | |

The aerosol device A according to the invention used for conditioning the above composition comprises the following characteristics:
- a valve equipped with a nozzle with an orifice 0.4 mm in size and an internal restriction orifice 1.6 mm in size, without an additional gas intake orifice,
- a slit diffuser for producing a fan-shaped spray, of "oil" nozzle type, from the company Lindal, this nozzle having a square orifice of 0.28×0.28 mm, opening out as a rectangular orifice of 2.30×0.33 mm. Consequently, this slit has a ratio of its largest dimension to its smallest dimension, along perpendicular axes, equal to 6.97.

The aerosol device B outside the invention used for conditioning the above composition comprises the following characteristics:
- the same valve as that of device A, and
- a diffuser with a circular-orifice nozzle.

### Results:

### • Device A

When sprayed for one second at a distance of 20 cm onto an ethanol-sensitive paper positioned perpendicular to the device, device A according to the invention produces a substantially rectangular spray imprint.

### • Device B

When sprayed for one second at a distance of 20 cm onto an ethanol-sensitive paper positioned perpendicular to the device, device B, outside the invention, produces a substantially circular spray imprint.

### Sensory analysis:

A panel of experts then performed a comparative evaluation between devices A and B, giving a grade ranging from 0 to 50 for each of the properties listed in the table below.

Thus, for the ease of application to the roots, grade 0 corresponds to a composition that is difficult to apply, i.e. that is poorly, non-uniformly distributed, and grade 50 corresponds to a composition that is very easy to apply, with good distribution of the composition on the roots.

For the fixing, grade 0 corresponds to poor fixing, and grade 50 corresponds to very good fixing.

As regards the volume, grade 0 corresponds to a poor apparent volume of the head of hair as a whole, and grade 50 corresponds to a substantial gain in volume.

The averages of the grades obtained are as follows:

| | A | B |
|---|---|---|
| Ease of application to the roots | 40 | 30 |
| Fixing | 40 | 40 |
| Provision of volume | 40 | 30 |

These results show that the treatment with device A of the invention affords greater ease of application to the roots and more volume to the hairstyle, for the same fixing, when compared with the treatment with device B outside the invention.

## Claims

1. Aerosol device formed by:
- a container containing an aerosol composition comprising one or more propellants and, in an alcoholic or aqueous-alcoholic medium, one or more fixing polymers, and
- a means for dispensing the said aerosol composition comprising a diffuser equipped with a slit-shaped end orifice that produces a fan-shaped spray.

2. Device according to Claim 1, **characterized in that** the slit-shaped end orifice comprises a ratio of its largest dimension to its smallest dimension, along perpendicular axes, of greater than or equal to 2, even more preferentially greater than or equal to 5.

3. Device according to Claim 1 or 2, **characterized in that** the fixing polymer(s) are chosen from anionic, amphoteric and nonionic fixing polymers.

4. Device according to any one of the preceding claims, **characterized in that** the fixing polymer(s) are anionic and chosen from copolymers of acrylic acid or methacrylic acid or salts thereof and of acrylamide, copolymers of acrylic acid or methacrylic acid with a monoethylenic monomer, crotonic acid copolymers, copolymers of C₄-C₈ monounsaturated carboxylic acids or anhydrides, polyacrylamides comprising carboxylate groups, and homopolymers and copolymers comprising sulfonic groups.

5. Device according to Claim 4, **characterized in that** the fixing polymer(s) are anionic and chosen from methyl vinyl ether/monoesterified maleic anhydride copolymers, acrylic acid/ethyl acrylate/N-tert-butylacrylamide terpolymers, copolymers of methacrylic acid and of methyl methacrylate, vinyl acetate/vinyl tert-butylbenzoate/crotonic acid terpolymers and crotonic acid/vinyl acetate/vinyl neododecanoate terpolymers, copolymers of methacrylic acid and of ethyl acrylate, butyl acrylate/acrylic acid/methacrylic acid branched block polymers and vinylpyrrolidone/acrylic acid/lauryl methacrylate terpolymers.

6. Device according to Claim 3, **characterized in that** the polymer(s) are nonionic and chosen from polyalkyloxazolines, vinyl acetate homopolymers, vinyl acetate copolymers, homopolymers and copolymers of acrylic esters, acrylonitrile copolymers, styrene homopolymers, styrene copolymers, polyamides, vinyllactam homopolymers and vinyllactam copolymers.

7. Device according to any one of the preceding claims, **characterized in that** the fixing polymer(s) are present in concentrations ranging from 0.1% to 20% by weight, preferably from 1% to 15% by weight and even more preferentially from 3% to 10% by weight relative to the total weight of the aerosol composition.

8. Device according to any one of the preceding claims, **characterized in that** the fixing polymer(s) are in the form of a solution or dispersion in the alcoholic or aqueous-alcoholic medium, with a dynamic viscosity of greater than 3 cPs, preferably ranging from 3 to 20 cPs and even more preferentially from 4 to 10 cPs, before being placed in contact with the propellant, at a temperature of 25°C and at a shear rate of 1 s⁻¹.

9. Device according to any one of the preceding claims, **characterized in that** the propellant(s) are chosen from dimethyl ether, and optionally chlorinated and/or fluorinated volatile hydrocarbons, and mixtures thereof.

10. Device according to any one of the preceding claims, **characterized in that** the propellant(s) are present in a concentration ranging from 10% to 80% by weight, preferably from 20% to 75% by weight and even more preferentially from 30% to 70% by weight relative to the total weight of the aerosol composition.

11. Device according to any one of the preceding claims, **characterized in that** the aerosol composition contains one or more thickeners chosen from natural or synthetic, anionic, amphoteric, zwitterionic, nonionic or cationic, associative or non-associative polymeric thickeners, preferably from cellulose thickeners, guar gum and derivatives thereof, gums of microbial origin, and crosslinked homopolymers of acrylic acid or acrylamidopropanesulfonic acid.

12. Device according to any one of the preceding claims, **characterized in that** the aerosol composition contains one or more silicones, one or more non-silicone fatty substances, one or more acidifying or basifying agents, and mixtures thereof.

13. Process for treating and especially for shaping keratin fibres, in particular the hair, **characterized in that** it comprises the use of the device as defined in any one of Claims 1 to 12, and optionally comprises a step of exposing the roots.

14. Use of the device as defined in any one of Claims 1 to 12, for shaping keratin fibres, such as the hair.

15. Use according to Claim 14, for giving the hairstyle volume.

## Patentansprüche

1. Aerosolvorrichtung, gebildet durch:
- einen Behälter, der eine Aerosolzusammensetzung enthält, die ein oder mehrere Treibmittel und ein oder mehrere festigende Polymere in einem alkoholischen oder wässrig-alkoholischen Medium umfasst, und
- eine Einrichtung zum Ausgeben der Aerosolzusammensetzung, umfassend einen Zerstäuber, der mit einer schlitzförmigen Endöffnung versehen ist, die einen fächerförmigen Sprühnebel erzeugt.

2. Vorrichtung gemäß Anspruch 1, **dadurch gekennzeichnet, dass** die schlitzförmige Endöffnung ein Verhältnis ihrer größten Abmessung zu ihrer kleinsten Abmessung entlang senkrechter Achsen von größer oder gleich 2, bevorzugter größer oder gleich 5, aufweist.

3. Vorrichtung gemäß Anspruch 1 oder 2, **dadurch gekennzeichnet, dass** die festigenden Polymer(e) ausgewählt sind aus anionischen, amphoteren und nichtionischen festigenden Polymeren.

4. Vorrichtung gemäß einem der vorstehenden Ansprüche, **dadurch gekennzeichnet, dass** die festigenden Polymer(e) anionisch sind und ausgewählt sind aus Copolymeren von Acrylsäure oder Methacrylsäure oder Salzen davon und von Acrylamid, Copolymeren von Acrylsäure oder Methacrylsäure mit einem monoethylenischen Monomer, Krotonsäure-Copolymeren, Copolymeren von einfach ungesättigten C₄-C₈-Carbonsäuren oder - Anhydriden, Polyacrylamiden, die Carboxylatgruppen umfassen, und Homopolymeren und Copolymeren, die Sulfonsäuregruppen umfassen.

5. Vorrichtung gemäß Anspruch 4, **dadurch gekennzeichnet, dass** die festigenden Polymer(e) anionisch sind und ausgewählt sind aus Methylvinylether/monoverestertes-Maleinsäureanhydrid-Copolymeren, Acrylsäure/Ethylacrylat/N-tert-Butylacrylamid-Terpolymeren, Copolymeren von Methacrylsäure und von Methylmethacrylat, Vinylacetat/Vinyl-tert-butylbenzoat/Crotonsäure-Terpoylmeren und Crotonsäure/Vinylacetat/Vinylneododecanoat-Terpolymeren, Copolymeren von Methacrylsäure und von Ethylacrylat, verzweigten Butylacrylat/Acrylsäure/Methacrylsäure-Blockpolymeren und Vinylpyrrolidon/Acrylsäure/Laurylmethacrylat-Terpolymeren.

6. Vorrichtung gemäß Anspruch 3, **dadurch gekennzeichnet, dass** die Polymer(e) nichtionisch sind und ausgewählt sind aus Polyalkyloxazolinen, Vinylacetat-Homopolymeren, Vinylacetat-Copolymeren, Homopolymeren und Copolymeren von Acrylsäureestern, Acrylnitril-Copolymeren, Styrol-Homopolymeren, Styrol-Copolymeren, Polyamiden, Vinyllactam-Homopolymeren und Vinyllactam-Copolymeren.

7. Vorrichtung gemäß einem der vorstehenden Ansprüche, **dadurch gekennzeichnet, dass** die festigenden Polymer(e) in Konzentrationen in dem Bereich von 0,1 Gew.-% bis 20 Gew.-%, vorzugsweise von 1 Gew.-% bis 15 Gew.-% und noch bevorzugter von 3 Gew.-% bis 10 Gew.-% bezogen auf das Gesamtgewicht der Aerosolzusammensetzung vorhanden sind.

8. Vorrichtung gemäß einem der vorstehenden Ansprüche, **dadurch gekennzeichnet, dass** die festigenden Polymer(e) in der Form einer Lösung oder Dispersion in dem alkoholischen oder wässrig-alkoholischen Medium mit einer dynamischen Viskosität von größer als 3 cPs, vorzugsweise in dem Bereich von 3 bis 20 cPs und noch bevorzugter von 4 bis 10 cPs, vor Inkontaktbringen mit dem Treibmittel, bei einer Temperatur von 25 °C und einer Scherrate von 1 s⁻¹ vorliegen

9. Vorrichtung gemäß einem der vorstehenden Ansprüche, **dadurch gekennzeichnet, dass** die Treibmittel ausgewählt sind aus Dimethylether und gegebenenfalls chlorierten und/oder fluorierten flüchtigen Kohlenwasserstoffen und Gemischen davon.

10. Vorrichtung gemäß einem der vorstehenden Ansprüche, **dadurch gekennzeichnet, dass** die Treibmittel in einer Konzentration in dem Bereich von 10 Gew.-% bis 80 Gew.-%, vorzugsweise von 20 Gew.-% bis 75 Gew.-% und noch bevorzugter von 30 Gew.-% bis 70 Gew.-% bezogen auf das Gesamtgewicht der Aerosolzusammensetzung vorhanden sind.

11. Vorrichtung gemäß einem der vorstehenden Ansprüche, **dadurch gekennzeichnet, dass** die Aerosolzusammensetzung ein oder mehrere Verdickungsmittel ausgewählt aus natürlichen oder synthetischen, anionischen, amphoteren, zwitterionischen, nichtionischen oder kationischen, assoziativen oder nichtassoziativen polymeren Verdickungsmitteln, vorzugsweise aus Cellulose-Verdickungsmitteln, Guargummi und Derivaten davon, Gummis mit mikrobiellem Ursprung und vernetzten Homopolymeren von Acrylsäure oder Acrylamidopropansulfonsäure umfasst.

12. Vorrichtung gemäß einem der vorstehenden Ansprüche, **dadurch gekennzeichnet, dass** die Aerosolzusammensetzung ein oder mehrere Silicone, ein oder mehrere Nichtsilicon-Fettstoffe, ein oder mehrere Säuerungs- oder Basifizierungsmittel und Gemische davon umfasst.

13. Verfahren zum Behandeln und insbesondere zum Formen von Keratinfasern, insbesondere des Haars, **dadurch gekennzeichnet, dass** es die Verwendung der Vorrichtung gemäß einem der Anasprüche 1 bis 12 umfasst und gegebenenfalls einen Schritt von Exponieren der Wurzeln umfasst.

14. Verwendung der Vorrichtung gemäß einem der Ansprüche 1 bis 12 zum Formen von Keratinfasern, wie z. B. dem Haar.

15. Verwendung gemäß Anspruch 14, um der Frisur Volumen zu verleihen.

## Revendications

1. Dispositif aérosol constitué par :
- un récipient contenant une composition aérosol comprenant un ou plusieurs agents propulseurs et, dans un milieu alcoolique ou hydroalcoolique, un ou plusieurs polymères fixants, et
- un moyen de distribution de ladite composition aérosol comprenant un diffuseur muni d'un orifice terminal en forme de fente assurant un spray plan.

2. Dispositif selon la revendication 1, **caractérisé en ce que** l'orifice terminal en forme de fente comprend un rapport de sa plus grande dimension à sa plus petite dimension, selon des directions perpendiculaires, supérieur ou égal à 2, encore plus préférentiellement supérieur ou égal à 5.

3. Dispositif selon la revendication 1 ou 2, **caractérisé en ce que** le ou les polymères fixants sont choisis parmi les polymères fixants anioniques, amphotères et non ioniques.

4. Dispositif selon l'une quelconque des revendications précédentes, **caractérisé en ce que** le ou les polymères fixants sont anioniques et choisis parmi les copolymères d'acide acrylique ou méthacrylique ou leurs sels et d'acrylamide, les copolymères d'acide acrylique ou méthacrylique avec un monomère monoéthylénique, les copolymères d'acide crotonique, les copolymères d'acides ou d'anhydrides carboxyliques monoinsaturés en C₄-C₈, les polyacrylamides comportant des groupements carboxylates, les homopolymères et copolymères comprenant des groupements sulfoniques.

5. Dispositif selon la revendication 4, **caractérisé en ce que** le ou les polymères fixants sont anioniques et choisis parmi les copolymères méthylvinyléther/anhydride maléique monoestérifiés, les terpolymères acide acrylique/acrylate d'éthyle/N-tertiobutylacrylamide, les copolymères d'acide méthacrylique et de méthacrylate de méthyle, les terpolymères acétate de vinyle/tertiobutylbenzoate de vinyle/acide crotonique et les terpolymères acide crotonique/acétate de vinyle/néododécanoate de vinyle, les copolymères d'acide méthacrylique et d'acrylate d'éthyle, les polymères séquencés branchés acrylate de butyle/acide acrylique/acide méthacrylique et les terpolymères vinylpyrrolidone/acide acrylique/méthacrylate de lauryle.

6. Dispositif selon la revendication 3, **caractérisé en ce que** le ou les polymères sont non ioniques et choisis parmi les polyalkyloxazolines, les homopolymères d'acétate de vinyle, les copolymères d'acétate de vinyle, les homopolymères et copolymères d'esters acryliques, les copolymères d'acrylonitrile, les homopolymères de styrène, les copolymères de styrène, les polyamides, les homopolymères de vinyllactame, et les copolymères de vinyllactame.

7. Dispositif selon l'une quelconque des revendications précédentes, **caractérisé en ce que** le ou les polymères fixants sont présents dans des concentrations variant de 0,1 à 20 % en poids, de préférence de 1 à 15 % en poids, et encore plus préférentiellement de 3 à 10 % en poids, par rapport au poids total de la composition aérosol.

8. Dispositif selon l'une quelconque des revendications précédentes, **caractérisé en ce que** le ou les polymères fixants se trouvent sous la forme d'une solution ou d'une dispersion dans le milieu alcoolique ou hydroalcoolique, possédant une viscosité dynamique supérieure à 3 cPs, de préférence allant de 3 à 20 cPs, encore plus préférentiellement de 4 à 10 cPs, avant d'être mis en contact avec l'agent propulseur, ceci à la température de 25°C et à une vitesse de cisaillement de 1 s⁻¹.

9. Dispositif selon l'une quelconque des revendications précédentes, **caractérisé en ce que** le ou les agents propulseurs sont choisis parmi le diméthyléther, les hydrocarbures volatils, éventuellement chlorés et/ou fluorés, et leurs mélanges.

10. Dispositif selon l'une quelconque des revendications précédentes, **caractérisé en ce que** le ou les agents propulseurs sont présents à une concentration variant de 10 à 80 % en poids, de préférence de 20 à 75 % en poids, et encore plus préférentiellement de 30 à 70 % en poids, par rapport au poids total de la composition aérosol.

11. Dispositif selon l'une quelconque des revendications précédentes, **caractérisé en ce que** la composition aérosol contient un ou plusieurs agents épaississants choisis parmi les épaississants polymériques naturels ou synthétiques, anioniques, amphotères, zwittérioniques, non ioniques ou cationiques, associatifs ou non, de préférence parmi les agents épaississants cellulosiques, la gomme de guar et ses dérivés, les gommes d'origine microbienne, les homopolymères réticulés d'acide acrylique ou d'acide acrylamidopropanesulfonique.

12. Dispositif selon l'une quelconque des revendications précédentes, **caractérisé en ce que** la composition aérosol contient une ou plusieurs silicones, un ou plusieurs corps gras non siliconés, un ou plusieurs agents acidifiants ou alcalinisants, et leurs mélanges.

13. Procédé de traitement et, notamment de mise en forme, des fibres kératiniques, en particulier les cheveux, **caractérisé par le fait qu'**il comprend la mise en oeuvre du dispositif tel que défini à l'une quelconque des revendications 1 à 12, et comprend éventuellement une étape de découvrement des racines.

14. Utilisation du dispositif tel que défini à l'une quelconque des revendications 1 à 12 pour la mise en forme des fibres kératiniques, telles que les cheveux.

15. Utilisation selon la revendication 14, pour conférer du volume à la coiffure.
